# EUROPÄISCHE PATENTSCHRIFT

(11) **EP 1 406 688 B1**
(45) Veröffentlichungstag und Bekanntmachung des Hinweises auf die Patenterteilung: **19.09.2007**
(21) Anmeldenummer: 02758087.7
(22) Anmeldetag: 10.07.2002
(51) Int. Cl.: A61M 16/00, A61M 16/08, A61M 16/12, A61M 16/16, A61M 16/20

(54) **ATEMTHERAPIEGERAET ZUR FREIHALTUNG DES NATUERLICHEN ATEMWEGES EINES MENSCHLICHEN KOERPERS UND DESSEN VERWENDUNGSVERFAHREN ZUR VERHINDERUNG VON SCHNARCHGERAUESCHEN**
RESPIRATORY THERAPY DEVICE FOR KEEPING FREE THE NATURAL RESPIRATORY TRACT OF A HUMAN BODY AND THE USE THEREOF IN ORDER TO PREVENT THE SOUND OF SNORING
APPAREIL DE THÉRAPIE RESPIRATOIRE POUR MAINTENIR OUVERTES LES VOIES RESPIRATOIRES SUPÉRIEURES D'UNE PERSONNE ET PROCÉDÉ DE SON UTILISATION POUR SUPPRIMER LE BRUIT DE RONFLEMENT

(30) Priorität: 12.07.2001 DE 20111396 U
(43) Veröffentlichungstag der Anmeldung: 14.04.2004
(73) Patentinhaber: Hoffrichter GmbH, 19061 Schwerin (DE)
(72) Erfinder: HOFFRICHTER, Helmut, 19075 Pampow (DE)
(74) Vertreter: Jaap, Reinhard
(86) Internationale Anmeldenummer: PCT/DE2002/002523
(87) Internationale Veröffentlichungsnummer: WO 2003/006095

(56) Entgegenhaltungen:
- EP-A- 0 330 740
- US-A- 5 655 522
- US-A- 5 947 115

## Beschreibung

Die Erfindung betrifft ein Verfahren nach dem Oberbegriff des Anspruchs 1 und ein Atemtherapiegerät nach dem Oberbegriff des Anspruchs 2. Derartige Geräte werden zur Behandlung der obstruktiven Schlafapnoe eingesetzt. Eine andere Anwendung ist die Verhinderung des Schnarchens.
Zur Atemtherapie sind sogenannte CPAP-Geräte (CPAP = Continuous Positive Airway Pressure) zur Erzeugung eines positiven Atemwegsdrucks bekannt, *wie sie bei*spielsweise in der US 5 655 522 beschrieben sind. Diese Geräte stellen in einer Gesichtsmaske Atemluft·zur Verfügung deren Druck kontinuierlich gegenüber dem atmosphärischen Druck um einige hPa erhöht ist. Wenn ein Patient an dieser künstlichen Atmosphäre atmet, dann bleiben seine Atemwege durch den positiven Relativdruck zum Atmen weit genug aufgespannt. Ein CPAP-Gerät bewirkt somit lediglich eine "pneumatische Schienung" der Atemwege. Der Atemvorgang selbst beruht ausschließlich auf Eigenatmung.
Schnarchen ist ein Hinweis auf erschlaffte und eingeengte Atemwege. Bei hoher Strömungsgeschwindigkeit durch den Rachen sinkt dort der Luftdruck ,weil mit steigender Geschwindigkeit der Abstand zwischen den Lüftmolekülen größer wird. Infolgedessen kollabiert dort das erschlaffte Gewebe und versperrt den Luftweg ganz. Dadurch verschwindet der strömungsbedingte Unterdruck, die Atemwege richten sich auf und die Luft strömt wieder, bis sie eine bestimmte Geschwindigkeit erreicht. Das führt dann sogleich wieder zum nächsten Verschluss. Durch schnelle Folge von Verschluss und Öffnung der Atemwege entsteht das Schnarchgeräusch. Ein CPAP-Gerät erhöht den Luftdruck gegenüber dem atmosphärischen Normaldruck (Relativdruck) so weit, dass die strömungsbedingte Druckminderung keinen Verschluss mehr bewirken kann und verhindert dadurch das Entstehen eines Schnarchgeräusches. Wenn der dem Schnarchen zugrunde liegende Mechanismus einen bestimmten Schweregrad übersteigt, dann setzt auch der Atemantrieb aus. Jetzt liegt die behandlungsbedürftige Krankheit "Obstruktive Schlafapnoe" vor, von der mindestens 1% der Bevölkerung betroffen ist. Die Krankheit wird vorwiegend mit CPAP-Geräten behandelt. Mechanische Hilfsmittel oder operative Eingriffe sind weitere, aber untergeordnete Therapiemöglichkeiten.
Ein Nachteil bekannter CPAP-Geräte besteht darin, dass sie einen inneren Strömungswiderstand besitzen, den der Patient mit seiner Atemmuskulatur überwinden muss. Daraus ergibt sich eine zusätzliche Belastung, denn der Innenwiderstand des Gerätes addiert sich zum Atemwegswiderstand des Patienten.
Ein weiterer Nachteil besteht darin, dass die Luft beim Durchgang durch das Gerät angewärmt wird und dadurch ihre relative Feuchte sinkt. In der Folge entzieht die Luft den Schleimhäuten das Wasser. Da ausgetrocknete Schleimhäute nicht nur unangenehm sind, sondern auch ihre Abwehrfunktion verlieren, ist im Zusammenhang mit der Atemtherapie eine Luftbefeuchtung erforderlich. CPAP-Geräte werden deshalb mit einem integrierten Luftbefeuchter ausgestattet oder der Luftbefeuchter wird als Zusatzgerät zwischen CPAP-Gerät und Patient eingeschaltet.
Weitere Nachteile von CPAP-Geräten sind Betriebsgeräusch, Behinderung durch die Gesichtsmaske, hygienische Risiken und hoher Energieverbrauch.

Es ist aus der EP-A-0 330 740 auch ein künstliches Beatmungsgerät bekannt, das unter anderem auch die Schlafapnoe behandelt und das aus einer Glocke und einem Gebläse besteht, die beide über Zuleitungen miteinander verbunden sind In diesen Zuleitungen sind technische Ventile und Regeleinrichtungen zur Beeinflussung der Funktion des Gebläses eingebunden. Die Glocke wird auf den Brustkorb eines menschlichen Körpers gesetzt und innerhalb der Glocke ein rhythmischer Wechsel zwischen einem Unterdruck und einem Überdruck erzeugt Dieser Rhythmus ist auf den natürlichen Atemrhythmus des Menschen abgestimmt, sodass der Atemvorgang aktiv unterstützt wird.
Der wesentliche Nachteil dieses Beatmungsgerätes besteht darin, dass es zur Verhinderung von Schnarchgeräuschen nicht geeignet ist.

Der Erfindung liegt daher die Aufgabe zugrunde, ein gattungsgemäßes Verfahren und ein entsprechendes Atemtherapiegerät zu schaffen, das ein Atmen an freier Atmosphäre ermöglicht. Weitere Aufgaben sind die Abschaffung der Gesichtsmaske, die Reduzierung von Betriebslärm, die Verbesserung der Hygiene und die Verminde-rung des Energieverbrauchs.

Diese Aufgabe wird verfahrensseitig durch die kennzeichnenden Merkmale des Anspruchs 1 und vorrichtungsseitig durch die kennzeichnenden Merkmale des An spruchs 2 gelöst. Zweckmäßige Ausgestahungsmöglichkeiten des Atemtherapiegerätes ergeben sich aus den Unteransprüchen 3 bis 6.
Die Erfindung beseitigt die Nachteile des Standes der Technik.
Indem man den ganzen Körper oder vorzugsweise nur Teile des Körpers, beispielsweise die vordere weiche Halsregion, in eine Kammer bringt, die unter vermindertem Relativdruck steht, wird das Kollabieren der Atemwege im Rachenhereich verhindert, weil dann der höhere statische Druck der Normalatmosphäre die Atemwege schient. Im physikalischen Sinne handelt es sich bei diesem Verfahren um eine Umkehrung des nach dem Stand der Technik bekannten CPAP-Prinzips. Dadurch kann der Patient an Normalatmosphäre atmen und hat den Vorteil, dass die hinderliche
Atemmaske nicht mehr nötig ist. Da die Atemluft keine technischen Bauteile durchströmen muss, ist auch kein zusätzlicher Atemwiderstand vorhanden und die Atemmuskulatur wird nicht zusätzlich belastet. Durch das Atmen an Normalatmosphäre ist keine zusätzliche Atemluftbefeuchtung erforderlich und der Aufwand für einen Luftbefeuchter entfällt.

Da das erfindungsgemäße Atemtherapiegerät nicht mehr die Atemluft liefern, sondern nur noch den negativen Relativdruck in einer Kammer erhalten muss, entfällt auch der Atemschlauch zugunsten eines dünnen Absaugschlauches. Das Sauggebläse muss jedoch keine großen Luftmengen bewegen, sondern immer nur diejenige Menge, die infolge von Undichtheiten in den Unterdruckraum einströmt. Die erforderliche Leistung des Sauggebläses ist damit klein und kann auch von einer Batterie aufgebracht werden. Außerdem kann das ganze Gebläse recht klein gehalten werden, wodurch eine gute Schallisolation ermöglicht wird wird.

Vorteile ergeben sich auch im Sicherheitsbereich. Ein Ausfall des Gebläses führt nicht automatisch zu einer CO₂-Rückatmung. In einem derartigen Fall würden sich die Symptome der obstruktiven Schlafapnoe wieder einstellen, mehr könnte jedoch nicht geschehen. Hygienische Vorteile entstehen, weil keine Zubehörteile wie Schlauch und Maske von der Atemluft durchströmt werden und sich dadurch kein Kondenswasser mehr bilden kann. Der zeitliche Reinigungszyklus kann deshalb wesentlich vergrößert werden. Eine Nutzung des Gerätes durch verschiedene Patienten ist unbedenklich.

Da das Gerät einfach aufgebaut und kostengünstig herstellhar ist und mit seiner Anwendung keine Gefahren verbunden sind, kann es auch ohne ärztliche Verschreibung eingesetzt werden, beispielsweise um das Schnarchen zu verhindern. Dafür bekannte Vorrichtungen bestehen zumeist aus einem Gegenstand, der im Mund oder an den Zähnen getragen werden muss. Dabei können leicht Unfälle entstehen, wenn sich beispielsweise die benutzte Antischnarch-Spange löst und verschluckt wird.

Die Erfindung soll nachfolgend an einem Anwendungsbeispiel näher erläutert werden. Die zugehörige Figur zeigt den Aufbau einer einfachen Realisierungsmöglichkeit.
Die Beispielvorrichtung besteht aus einer Halsmaske 1, einem Absaugschlauch 2 und einer Saugpumpe 3. Die Halsmaske 1 ist eine halboffene Kammer, die kuppelförmig die vordere (ventrale) Halsregion überspannt. Der Rand der Halsmaske 1 umschreibt in einem Umlauf die Region vom Kinn ausgehend an beiden Seiten des Unterkiefers entlang zu den Seiten des Halses und von dort wieder nach vorn unten bis zum oberen Ende des Brustbeins. Entlang des Umlaufs ist eine Dichtvorrichtung 6 angeordnet, die im Gebrauchszustand auf der Haut aufliegt. Auf diese Weise wird die offene Seite der Halsmaske 1 von der Haut des Patienten verschlossen und ein abgeschlossenes Kammervolumen 7 ausgebildet.
Die Oberfläche der Halsmaske ist so beschaffen, dass sie senkrecht zu ihrer Oberfläche steif, jedoch in Ausdehnungsrichtung der Oberfläche flexibel ist. Dadurch kann sich die Maskenoberfläche bei Nickbewegungen des Kopfes vergrößern und verkleinern und bei Drehbewegungen des Kopfes seitlich verziehen. Im Beispiel wird diese Eigenschaft dadurch erreicht, dass die Hülle der Halsmaske 1 aus einer elastischen Haut 5, beispielsweise aus Gummi oder einem anderen Elastomer über einem Unterbau aus stabilisierenden Skelettspangen 4 besteht.

An einer geeigneten Stelle, vorzugsweise im unteren Bereich der Halsmaske 1 ist eine stutzenförmige Luftausleitung 8 angeordnet, die von außen durch die Hülle der Halsmaske 1 hindurch in das abgeschlossenes Kammervolumen 7 führt und die über den Absaugschlauch 2 mit der Saugseite der Saugpumpe 3 verbunden ist.
Zum Anlegen und zur Stabilisierung des korrekten Sitzes der Halsmaske 1 ist ein Nackenhand 9 vorgesehen, das auch mit einem Verschluss, vorzugsweise mit einem Klettverschluss versehen werden könnte.

Während der Benutzung erzeugt die Saugpumpe 3 über den an der Luftausleitung 8 angeschlossenen Absaugschlauch 2 in dem abgeschlossenen Kammervolumen 7 einen konstanten Unterdruck von wenigen hPa. Das wird durch eine druckgeregelte Absaugpumpe oder durch Einstellung einer bestimmten Drehzahl an einem Sauggebläse erreicht. Sofern Leckagen zwischen der Haut des Patienten und der Dichtvorrichtung 6 entstehen, wird das in das abgeschlossene Kammervolumen 7 einströmende Leckvolumen abgesaugt. Auch Kopfbewegungen bewirken keine Änderung des Unterdruckes in dem abgeschlossenen Kammervolumen 7, wenn die Saugpumpe 3 oder das verwendete Sauggebläse eine Vorrichtung zur Druckregelung aufweisen.

Da die Halsmaske 1 die gesamte ventrale Halsregion überspannt und an dieser Stelle mit dem abgeschlossenen Kammervolumen 7 eine künstliche Unterdruckatmosphäre herstellt, werden die Atemwege des Patienten durch den Druck der Normalatmosphäre geschient. Strömungbedingte Druckabsenkungen im Rachenbereich des Patienten führen nicht mehr zum Kollabieren der Atemwege. Schnarchgeräusche können nicht mehr entstehen. Patienten mit einer obstruktiven Schlafapnoe können frei atmen und haben wieder einen gesunden Schlaf.

### Aufstellung der verwendeten Bezugszeichen

- 1: druckstabile Kappe
- 2: Absaugschlauch
- 3: Saugpumpe
- 4: Skelettspangen
- 5: Oberfläche der Kammer
- 6: Dichtvorrichtung
- 7: Unterdruckkammer
- 8: Luftausleitung
- 9: Nackenband

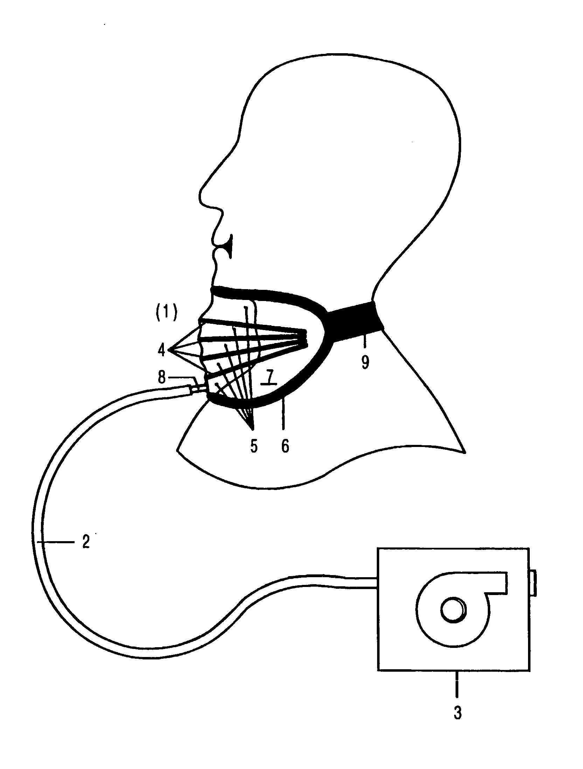

## Patentansprüche

1. Verfahren zur Verhinderung von Schnarchgeräuschen, bei dem die oberen Atemwege eines Menschen ständig offen gehalten werden,
**dadurch gekennzeichnet, dass** der Atemweg dem Druck der Atmosphäre und der extrakorporale Bereich des Atemweges einem künstlichen Unterdruck ausgesetzt werden, wobei zwischen dem inneren Atmosphärendruck und dem äußeren Unterdruck eine kontinuierliche Differenz besteht.

2. Atemtherapiegerät zur Freihaltung des natürlichen Atemweges eines menschlichen Körpers, bestehend aus einer Pumpe und einer Zuleitung zur Erzeugung eines kontinuierlichen Unterdruckes,
**dadurch gekennzeichnet, dass** die Pumpe eine Saugpumpe (3) ist, die über einen Absaugschlauch (2) mit einem druckstabilen Hohlkörper verbunden ist, der den menschlichen Körper unter Freihaltung der natürlichen Atemöffnung umgibt und der zumindest im extrakorporalen Bereich des Atemweges eine Unterdruckkammer (7) ausbildet.

3. Atemtherapiegerät nach Anspruch 2,
**dadurch gekennzeichnet, dass** der druckstabile Hohlkörper als eine Kappe (1) ausgebildet ist, die mit ihrem Rand die vordere Halsregion vom Kinn ausgehend an beiden Seiten des Unterkiefers, der Halsseiten und der Schlüsselbeingegend entlang bis zum oberen Ende des Brustbeins umläuft und so ausgeformt ist, dass sie den von ihrem Rand eingegrenzten Hautbereich kuppelförmig mit einem Abstand zur Haut überdeckt.

4. Atemtherapiegerat nach Anspruch 3,
**dadurch gekennzeichnet, dass** die druckstabile Kappe (1) aus einem Material besteht, das senkrecht zur Körperoberfläche druckstabil ist und in Oberflächenrichtung so flexibel ist, dass Bewegungen des Kopfes zugelassen sind.

5. Atemtherapiegerät nach Anspruch 4,
**dadurch gekennzeichnet, dass** die druckstabile Kappe (1) aus einem vorzugsweise integrierten Unterbau aus stabilisierenden Skelettstangen (4) und einer darüber liegenden elastische Haut 5, beispielsweise aus Gummi oder einem anderen Elastomer, besteht.

6. Atemtherapiegerät nach Anspruch 1,
**dadurch gekennzeichnet, dass** die Saugpumpe (3) mit einer Druckregeleinrichtung zur Erzeugung und Aufrechtsrhaltung eines voreinstellbaren Unterdruckes ausgerüstet ist.

## Claims

1. Method for preventing snoring noises, in which the upper respiratory tract of a human is constantly held open, **characterised in that** the respiratory tract is subjected to the pressure of the atmosphere and the extracorporeal area of the respiratory tract to an artificial negative pressure, wherein there is a continuous difference between the inner atmospheric pressure and the outer negative pressure.

2. Respiratory therapy device for keeping free the natural respiratory tract of a human body, consisting of a pump and a feed line for generating a continuous negative pressure, **characterised in that** the pump is a suction pump (3) which is connected via a suction tube (2) to a pressure-stable hollow body which surrounds the human body, while keeping free the natural respiratory opening, and which forms a negative pressure chamber (7) at least in the extracorporeal area of the respiratory tract.

3. Respiratory therapy device according to Claim 2, **characterised in that** the pressure-stable hollow body is formed as a cap (1) which extends with its edge around the front neck area from the chin along both sides of the lower jaw, the sides of the neck and the collarbone region as far as the upper end of the breastbone and is shaped such that it covers the skin area which is enclosed by its edge like a dome at a spacing from the skin.

4. Respiratory therapy device according to Claim 3, **characterised in that** the pressure-stable cap (1) consists of a material which is pressure-stable perpendicularly to the surface of the body and is so flexible in the direction of the surface that movements of the head are allowed.

5. Respiratory therapy device according to Claim 4, **characterised in that** the pressure-stable cap (1) consists of a preferably integrated substructure of stabilising skeleton ribs (4) and a resilient skin (5), for example of rubber or another elastomer, lying on top.

6. Respiratory therapy device according to Claim 1, **characterised in that** the suction pump (3) is fitted with a pressure regulating device for generating and maintaining a negative pressure which can be preset.

## Revendications

1. Procédé pour éviter les bruits de ronflement, en maintenant les voies respiratoires supérieures d'une personne ouvertes en permanence,
**caractérisé en ce qu'**
on soumet les voies respiratoires à la pression atmosphérique et la zone extracorporelle des voies respiratoires à une dépression artificielle, avec une différence continue entre la pression atmosphérique intérieure et la dépression extérieure.

2. Appareil de thérapie respiratoire pour maintenir libres les voies respiratoires naturelles d'une personne, constitué d'une pompe et d'une conduite d'arrivée pour générer une dépression continue,
**caractérisé en ce que**
la pompe est une pompe d'aspiration (3) reliée par un tuyau d'aspiration (2) à un corps creux stable en pression qui entoure le corps humain en libérant l'orifice respiratoire naturel, et qui forme, au moins dans la zone extracorporelle des voies respiratoires, une chambre de dépression (7).

3. Appareil de thérapie respiratoire selon la revendication 2,
**caractérisé en ce que**
le corps creux stable en pression est en forme d'un capuchon (1) dont le bord entoure la région avant de la gorge en partant du menton, au niveau des deux côtés de la mâchoire inférieure, le long des côtés de la gorge et de la région de la clavicule, jusqu'à l'extrémité supérieure du sternum, et formé de telle sorte qu'il recouvre la zone de la peau adjacente à son bord en forme de coupole, à une certaine distance de la peau.

4. Appareil de thérapie respiratoire selon la revendication 3,
**caractérisé en ce que**
le capuchon stable en pression (1) se compose d'un matériau exerçant une pression constante perpendiculairement à la surface du corps, et suffisamment flexible dans la direction de la surface pour permettre des mouvements de la tête.

5. Appareil de thérapie respiratoire selon la revendication 4,
**caractérisé en ce que**
le capuchon stable en pression (1) se compose d'une sous-structure de préférence intégrée faite de tiges d'ossature stabilisantes (4) et d'une peau élastique (5) située par-dessus, par exemple en caoutchouc ou en un autre élastomère.

6. Appareil de thérapie respiratoire selon la revendication 1,
**caractérisé en ce que**
la pompe d'aspiration (3) est équipée d'un dispositif de régulation de pression pour générer et maintenir une dépression préréglée.
